# EUROPEAN PATENT SPECIFICATION

(11) **EP 4 261 227 B1**
(45) Date of publication and mention of the grant of the patent: **16.09.2026**
(21) Application number: 22871058.8
(22) Date of filing: 23.12.2022
(51) Int. Cl.: C07K 16/40, C12N 15/13, C12N 5/20, G01N 33/573, G01N 33/577

(54) **ANTI-OXACILLINASE-48-LIKE(OXA-48-LIKE) CARBAPENEMASE MOUSE HYBRIDOMA CELL LINE, MAB, AND APPLICATION**
HYBRIDOMZELLINIE DER ANTI-OXACILLINASE-48-LIKE (OXA-48-LIKE)-CARBAPENEMASE- MAUS, MONOKLONALEN ANTIKÖRPER UND ANWENDUNG
SOUCHE CELLULAIRE D'HYBRIDOME ANTI-OXA-48 CARBAPÉNÉMASE DE SOURIS, ANTICORPS MONOCLONAL ET UTILISATION

(30) Priority: 01.03.2022 CN 202210189253
(43) Date of publication of application: 18.10.2023
(73) Proprietor: Tianjin Era Biology Technology Co., Ltd., Tianjin 300457 (CN); BEIJING GOLD MOUNTAINRIVER TECH DEVELOPMENT CO., LTD., Beijing 100081 (CN); Genobio Pharmaceutical Co., Ltd., Binhai New Area Tianjin 300480 (CN)
(72) Inventor: YUAN, Qinghua, Tianjin 300480 (CN); LI, Keke, Tianjin 300480 (CN); HE, Yongsheng, Tianjin 300480 (CN); CHEN, Xiaoling, Tianjin 300480 (CN); ZHOU, Yuehui, Tianjin 300480 (CN); FAN, Linlin, Tianjin 300480 (CN); LIU, Qian, Tianjin 300480 (CN); WANG, Yamiao, Tianjin 300480 (CN); KONG, Di, Tianjin 300480 (CN); WANG, Siyi, Tianjin 300480 (CN)
(74) Representative: Bayramoglu et al.
(86) International application number: PCT/CN2022/141242
(87) International publication number: WO 2023/165229

(56) References cited:
- EP-A1- 4 063 497
- EP-A1- 4 063 498
- WO-A1-2016/092096
- WO-A1-2020/016814
- CN-A- 112 500 489
- CN-A- 112 501 131
- CN-A- 112 980 803
- CN-A- 112 980 804
- CN-A- 114 317 454
- BOUTAL HERVÉ ET AL: "A multiplex lateral flow immunoassay for the rapid identification of NDM-, KPC-, IMP- and VIM-type and OXA-48-like carbapenemase-producing Enterobacteriaceae", JOURNAL OF ANTIMICROBIAL CHEMOTHERAPY, vol. 73, no. 4, 22 January 2018 (2018-01-22), GB, pages 909 - 915, XP055841261, ISSN: 0305-7453, DOI: 10.1093/jac/dkx521
- KON HADAS ET AL: "Multiplex lateral flow immunochromatographic assay is an effective method to detect carbapenemases without risk of OXA-48-like cross reactivity", vol. 20, no. 1, 4 September 2021 (2021-09-04), GB, XP093140411, ISSN: 1476-0711, Retrieved from the Internet <URL:https://link.springer.com/article/10.1186/s12941-021-00469-0/fulltext.html> DOI: 10.1186/s12941-021-00469-0
- YOURI GLUPCZYNSKI ET AL: "Evaluation of two new commercial immunochromatographic assays for the rapid detection of OXA-48 and KPC carbapenemases from cultured bacteria", JOURNAL OF ANTIMICROBIAL CHEMOTHERAPY, vol. 71, no. 5, 28 January 2016 (2016-01-28), GB, pages 1217 - 1222, XP055282625, ISSN: 0305-7453, DOI: 10.1093/jac/dkv472
- BIOTECH NG: "NG-Test CARBA 5 - Rapid test for the detection of carbapenemases KPC, OXA-48-like, VIM, IMP and NDM in a bacterial colony from culture For professional in vitro diagnostic use only", 1 June 2020 (2020-06-01), XP093312448, Retrieved from the Internet <URL:https://biotrading.com/wp-content/uploads/2020/06/ng-test-carba5.pdf>
- FERNANDO PASTERAN ET AL: "Rapid Identification of OXA-48 and OXA-163 Subfamilies in Carbapenem-Resistant Gram-Negative Bacilli with a Novel Immunochromatographic Lateral Flow Assay", JOURNAL OF CLINICAL MICROBIOLOGY, vol. 54, no. 11, 1 November 2016 (2016-11-01), US, pages 2832 - 2836, XP055716255, ISSN: 0095-1137, DOI: 10.1128/JCM.01175-16

## Description

### TECHNICAL FIELD

The present invention belongs to the technical field of antibody preparation, in particular to an anti-oxacillinase-48-like (OXA-48-like) carbapenemase mouse hybridoma cell line, a mAb, and an application.

### BACKGROUND

Carbapenemases are β-lactamases with a high ability to hydrolyze carbapenem antibiotics. According to the Ambler molecular structure, carbapenemases are divided into classes A, B, and D enzymes. Class A enzymes include *Klebsiella pneumonia* Carbapenemase (KPC). Class B enzymes include New Delhi Metallo-beta-lactamase (NDM), imipenemase (IMP), Verona integron-encoded Metallo-β-lactamase (VIM), and German imipenemase (GIM). Class D enzymes include oxacillinase (OXA). Specifically, OXA-48-like enzymes play an increasingly important role in carbapenem resistance, and there are a growing number of reports on carbapenem-resistant *Enterobacteriaceae* worldwide. In 2001, Poirel L et al. discovered a new β-lactamase gene, i.e., blaOXA-48, from a *Klebsiella pneumonia* strain resistant to almost all β-lactam drugs. Since the discovery of OXA-48, the number of OXA-48-producing bacteria detected has been constantly rising in the Mediterranean area in the past decade. Subsequently, OXA-48 has been reported in Mexico, Libya, Egypt, Tunisia, India, and other countries. In recent years, OXA-48 has also emerged in the United States and Taiwan.

There are many methods for the laboratory detection of carbapenemases, mainly including the modified Hodge test, Carba NP test, modified carbapenem inactivation method (mCIM), enzyme inhibitor enhancement test, immune-gold labeling test, molecular biological method, and the like. Immunodiagnostic reagents are the fastest-growing subdivision of *in vitro* diagnostic reagents, which implement qualitative or quantitative detection based on the specific binding between antigens and antibodies. The development of carbapenemase rapid diagnostic products with independent intellectual property rights is of great significance for the early typing of drug-resistant strains, the guidance of drug administration, and the improvement of medicine and health in China. Boutal et al. J Antimicrob Chemother 2018; 73: 909-915 and Kon et al. Ann Clin Microbiol Antimicrob (2021) 20:61 disclose the detection of OXA-48-like carbapenemases in a lateral flow immunoassay (LFIA) that uses mAb pairs for capture and detection; this test is known in the art as CARBA 5. Glupczynski et al J Antimicrob Chemother 2016; 71: 1217-1222 refers to an immunochromatographic assay known in the art as the OXA-48 K-SeT that also relies on binding to two epitopes specific to OXA-48-like carbapenemases by capture and detection murine mAbs, using colloidal gold nanoparticles bound to a nitrocellulose membrane within a lateral flow device.

### SUMMARY

In order to solve the above technical problems, the present invention provides technical solutions defined by the appended claims. The technical information set out below may in some respects go beyond the disclosure of the claimed invention, which is defined exclusively by the appended claims. The additional technical information is provided to place the invention in a broader technical context and to illustrate possible related technical developments. Such additional technical information, which does not fall within the scope of the appended claims, is not part of the invention.

The advantages and positive effects of the present invention are as follows: the present invention provides two anti-OXA-48-like carbapenemase mouse hybridoma cell lines, which can respectively produce two anti-OXA-48-like carbapenemase mouse mAbs. After systematic evaluation, including evaluations on the antibody subtype and titer, kit sensitivity, specificity, and stability, It is found that the anti-OXA-48-like carbapenemase mouse mAbs have good performance in all aspects and the titer reaches more than 1:1280000, thereby the anti-OXA-48-like carbapenemase mouse mAbs are suitable for use as an immunodiagnostic reagent in the preparation of an *in vitro* diagnostic kit for carbapenemase resistant strains.

### BRIEF DESCRIPTION OF THE DRAWINGS

FIG. 1 is an image showing the protein electrophoresis of an OXA-48-like carbapenemase;
FIG. 2 is an image showing the protein electrophoresis of anti-OXA-48-like carbapenemase mouse mAbs;
FIG. 3 shows the detection results of an OXA-48-like carbapenemase test card by a colloidal gold method.

Biological material: IB5 was deposited in China General Microbiological Culture Collection Center (CGMCC) on August 27, 2020 with the accession number of CGMCC No. 20284;

Biological material: IH3 was deposited in CGMCC on August 27, 2020 with the accession number of CGMCC No. 20283.

### DETAILED DESCRIPTION OF THE EMBODIMENTS

The embodiments of the present invention are explained below.

The present invention relates to an anti-OXA-48-like carbapenemase mouse hybridoma cell line, the biological material of which is named IB5 and belongs to the hybridoma cell. IB5 was deposited in CGMCC on August 27, 2020 with the accession number of CGMCC No. 20284 and is tested to be alive. Another anti-OXA-48-like carbapenemase mouse hybridoma cell line is further provided, the biological material of which is named IH3 and belongs to the hybridoma cell. IH3 was deposited in CGMCC on August 27, 2020 with the accession number of CGMCC No. 20283 and is tested to be alive.

The antibody IB5 produced by the anti-OXA-48-like carbapenemase mouse hybridoma cell line includes a light chain variable region and a heavy chain variable region. The light chain variable region includes CDRL1 as shown in SEQ ID NO: 1, CDRL2 as shown in SEQ ID NO: 2, and CDRL3 as shown in SEQ ID NO: 3. The heavy chain variable region includes CDRH1 as shown in SEQ ID NO: 4, CDRH2 as shown in SEQ ID NO: 5, and CDRH3 as shown in SEQ ID NO: 6.
SEQ ID NO: 1 RASKSISKYLA (CDRL1)
SEQ ID NO: 2 SGSTFQS (CDRL2)
SEQ ID NO: 3 QQHNEYPYVT (CDRL3)
SEQ ID NO: 4 GFTFSTYAMS (CDRH1)
SEQ ID NO: 5 TISSGGSYTSYPDSVKG (CDRH2)
SEQ ID NO: 6 RPEYAMDY (CDRH3);

The amino acid sequence of the light chain variable region of the antibody IB5 is shown in SEQ ID NO: 7, and the amino acid sequence of the heavy chain variable region of the antibody IB5 is shown in SEQ ID NO: 9;
SEQ ID NO: 7 
DWIQLVQHQPVSSYLAASPGETVTINCRASKSISKYLAWYQEKPGKTNK
LLIYSGSTFQSGFPSGFSGSGSGTDFTLTVSSLEPEDFAIYYCQQHNEYPYVTFG
GGTKLEIKRA
**SEQ ID** NO: 9

QCEVMLVESGGGLVKPGGSLKLSCAASGFTFSTYAMSWVRLTPEKRLE
WVATISSGGSYTSYPDSVKGRFTISRDNAKNTLYLQMSSLRSEDTAMYYCAR
RPEYAMDYWGQGTSVTVSSA;

The nucleotide sequence encoding the light chain variable region of the antibody IB5 is shown in SEQ ID NO: 8, and the nucleotide sequence encoding the heavy chain variable region of the antibody IB5 is shown in SEQ ID NO: 10;
SEQ ID NO: 8 
GATTGGATACAGTTGGTGCAGCATCAGCCCGTTTCATCTTATCTTGCT
GCATCTCCTGGAGAAACCGTTACTATTAATTGCAGGGCAAGTAAGAGCAT
TAGCAAGTATTTAGCCTGGTATCAAGAGAAACCTGGGAAAACTAATAAGC
TTCTTATCTACTCTGGTTCCACTTTCCAATCTGGATTTCCATCAGGGTTCAG
TGGCAGTGGATCTGGTACAGATTTCACTCTCACCGTCAGTAGCCTGGAGC
CTGAAGACTTTGCAATCTATTACTGTCAACAGCATAATGAGTACCCGTAC
GTCACGTTCGGAGGGGGGACCAAGCTGGAGATAAAACGGGCT
SEQ ID NO: 10

CAGTGTGAAGTGATGCTGGTGGAGTCTGGGGGAGGCTTAGTGAAGCC
TGGAGGGTCCCTGAAACTCTCCTGTGCAGCCTCTGGATTCACTTTCAGTAC
CTATGCCATGTCTTGGGTTCGCCTGACTCCGGAGAAGAGGCTGGAGTGGG
TCGCAACCATTAGTAGTGGTGGTAGTTACACCTCCTATCCAGACAGTGTG
AAGGGGCGCTTCACCATCTCCAGAGACAATGCCAAGAACACCCTGTACCT
GCAAATGAGCAGTCTGAGGTCTGAGGACACGGCCATGTATTACTGTGCAC

GACGACCCGAATATGCTATGGACTACTGGGGTCAAGGAACCTCAGTCACC
GTCTCCTCAGCC;

The antibody IH3 produced by the anti-OXA-48-like carbapenemase mouse hybridoma cell line is provided, the light chain variable region of which includes CDRL1 as shown in SEQ ID NO: 11, CDRL2 as shown in SEQ ID NO: 12, and CDRL3 as shown in SEQ ID NO: 13, the heavy chain variable region of which includes CDRH1 as shown in SEQ ID NO: 14, CDRH2 as shown in SEQ ID NO: 15, and CDRH3 as shown in SEQ ID NO: 16;
SEQ ID NO: 11 RASKSVSTSGYSYMH (CDRL1)
SEQ ID NO: 12 LVSNLES (CDRL2)
SEQ ID NO: 13 QHIRELYT (CDRL3)
SEQ ID NO: 14 GYTFTDYVIS (CDRH1)
SEQ ID NO: 15 EIYPGSDTTYHNEKFKG (CDRH2)
SEQ ID NO: 16 DYGSNYGWFAY (CDRH3).

The amino acid sequence of the light chain variable region of the antibody IH3 is shown in SEQ ID NO: 17, and the amino acid sequence of the heavy chain variable region of the antibody IH3 is shown in SEQ ID NO: 19;
SEQ ID NO: 17 
DIVLTQSPASLAVSLGQRATISYRASKSVSTSGYSYMHWNQQKPGQPPR
LLIYLVSNLESGVPARFSGSGSGTDFTLNIHPVEEEDAATYYCQHIRELYTFGG
GTKLEIK
SEQ ID NO: 19

EVKLQQSGPELVKPGASVKMSCMASGYTFTDYVISWVKQRNGQGLEWI
GEIYPGSDTTYHNEKFKGKATLTADISSNTVYMQLSSLTFEDSAVYFCARDYG
SNYGWFAYWGQGTLVTVSA.

The nucleotide sequence encoding the light chain variable region of the antibody IH3 is shown in SEQ ID NO: 18, and the nucleotide sequence encoding the heavy chain variable region of the antibody IH3 is shown in SEQ ID NO: 20;
SEQ ID NO: 18 
GACATTGTGCTGACACAGTCTCCTGCTTCCTTAGCTGTATCTCTGGGG
CAGAGGGCCACCATCTCATACAGGGCCAGCAAAAGTGTCAGTACATCTGG
CTATAGTTATATGCACTGGAACCAACAGAAACCAGGACAGCCACCCAGAC
TCCTCATCTATCTTGTATCCAACCTAGAATCTGGGGTCCCTGCCAGGTTCA
GTGGCAGTGGGTCTGGGACAGACTTCACCCTCAACATCCATCCTGTGGAG
GAGGAGGATGCTGCAACCTATTACTGTCAGCACATTAGGGAGCTTTACAC
GTTCGGAGGGGGGACCAAGCTGGAAATAAAA
SEQ ID NO: 20

GAGGTGAAGCTGCAGCAGTCTGGACCTGAGTTGGTGAAGCCTGGGGC
TTCAGTGAAGATGTCCTGCATGGCTTCTGGATACACATTCACTGACTATGT
TATAAGTTGGGTGAAGCAGAGAAATGGACAGGGCCTTGAGTGGATTGGA
GAGATTTATCCTGGAAGTGATACCACTTACCACAATGAGAAATTCAAGGG
CAAGGCCACACTGACTGCAGACATATCCTCCAACACAGTCTACATGCAGC
TCAGTAGTCTGACATTTGAGGATTCTGCGGTCTATTTCTGTGCAAGAGACT
ACGGTAGTAACTACGGCTGGTTTGCTTACTGGGGCCAAGGGACTCTGGTC
ACTGTCTCTGCA.

The systematic evaluation of the anti-OXA-48-like carbapenemase mouse mAbs was performed, including evaluations on the antibody subtype and titer, kit sensitivity, specificity, and stability, which showed that the anti-OXA-48-like carbapenemase mouse mAbs had good performance in all aspects and were suitable for use as an immunodiagnostic reagent in the preparation of *in vitro* diagnostic kits. The anti-OXA-48-like carbapenemase mouse mAb can be made into colloidal gold immunoassay kits, chemiluminescence kits, radioimmunoassay kits, enzyme-linked immunoassay kits, fluorescence immunoassay kits, or microfluidic chips. The kits prepared can be used to detect an OXA-48-like carbapenemase antigen. The two antibodies involved in the present solution are especially suitable for making a double-antibody sandwich immuno-colloidal gold test strip, where the antibody IB5 is used as a coated antibody, and the antibody IH3 is used as a gold-labeled antibody. The produced double-antibody sandwich immuno-colloidal gold test strip is more sensitive. Similarly, the antibody IB5 can also be used as the gold-labeled antibody, and the antibody IH3 can be used as the coated antibody. The present invention is further explained by specific embodiments below. Specifically, the experimental methods where operation steps are not specified are carried out in accordance with the corresponding product specifications. The instruments, reagents, and consumables used in the embodiments can be purchased from commercial companies unless otherwise specified.

### Embodiment 1: Preparation of the anti-OXA-48-like carbapenemase mouse mAb

### 1.1 Antigen expression

A recombinant plasmid was obtained: An OXA-48 gene sequence was constructed on a Pet-28a (+) vector, and a whole gene was synthesized.

Transformation into host bacteria: The vector Pet-28a (+) containing a target gene was transformed into a cloned host *Escherichia coli (E. coli).*

Expanded culture: Transformants that were verified correctly were subjected to expanded culture, transferred to a kanamycin-resistant LB liquid medium, and subjected to shaking culture in a shaker at 37°C.

Induction: When an optical density (OD) value of a bacterial solution was between 0.4 and 0.6, isopropyl β-d-thiogalactoside (IPTG) was added until a final concentration was 0.1 mM. The bacteria were incubated at 30°C for 4 h and collected by centrifugation.

Ultrasonic crushing of the bacteria: About 1 g of the bacteria were added to 30 mL of phosphate-buffered saline (PBS) for re-suspension and then ultrasonically crushed at a power of 400 W for the first time for 3 s and for the next time after an interval of 5 s. After about 30 min, the bacterial solution was not sticky and was clarified, the bacterial solution was centrifuged to remove bacterial debris, and the resulting supernatant was filtered through a 0.45 µm filter film.

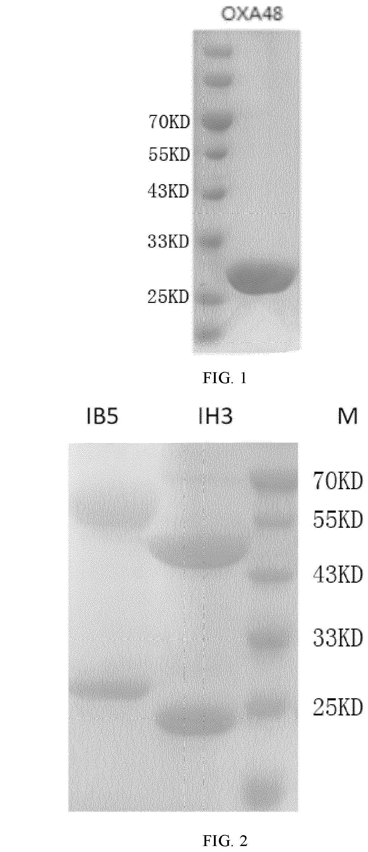

Purification: The supernatant was purified by a nickel column and then dialyzed into PBS to obtain a target protein with high purity, where the molecular weight was consistent with the predicted molecular weight of 30.7 kDa. Sodium dodecyl sulfate polyacrylamide gel electrophoresis (SDS-PAGE) is shown in FIG. 1, and the target protein was quantified by a bicinchoninic acid (BCA) method and sub-packaged for subsequent experiments.

### 1.2 Mouse immunity

Female BALB/c mice, aged about six weeks, were immunized with the purified OXA-48-like carbapenemase for antibody preparation, and the protein content was 0.1 mg/mL. According to the immunization dose, the mice were divided into two groups, with 5 mice in each group. According to the antigen content, the immunization dose of the first group was 25 µg/mouse, and the immunization dose of the second group was 50 µ g/mouse. For the first immunization, an appropriate amount of the OXA-48-like carbapenemase was diluted to 300 µL with distilled water, the same amount of 300 µL of Freund's complete adjuvant was added for thorough emulsification, and the mice were immunized by subcutaneous injection at multiple points. Two weeks later, the second immunization was carried out by intraperitoneal injection with the same dose. Two weeks later, the mice were immunized again by intraperitoneal injection. Seven days later, blood was collected from the mouse tail, and the enzyme-linked immunosorbent assay (ELISA) was used to measure the mouse serum titer.

The specific procedure was as follows: The ELISA plates were subjected to coating overnight at 4°C with 100 µ L of 0.2 µ g/mL OXA-48 per well, and the resulting ELISA plates were shaken for drying and washed 3 times with phosphate buffered saline with tween-20 (PBST). The resulting ELISA plates were blocked at 37°C for 2 h with 200 µL of 5% skimmed milk powder per well. Blood was collected from the mouse tail and centrifuged at 3000 r/min, and serum was collected after centrifugation and doubling diluted in PBS from 1:1000 to 1:512000 for later use. The resulting ELISA plates were shaken for drying and washed 3 times with PBST. The primary antibody diluted in PBS was added in the resulting ELISA plates from 1: 1000, with 100 µL per well, and incubated at 37°C for 1 h. The resulting ELISA plates were shaken for drying and washed 3 times with PBST. The goat anti-mouse secondary antibody diluted in PBS at 1:6000 was added in the resulting ELISA plates, with 100 µL per well, and incubated at 37°C for 45 min. The resulting ELISA plates were shaken for drying and washed 5 times with PBST. 3,3',5,5'-tetramethylbenzidine (TMB) was added in the resulting ELISA plates, with 100 µL per well. Color development was performed at 37°C for 10 min, the reaction was terminated, and values were read.

### 1.3 Cell fusion

Three days before fusion, the mice were immunized with the same vaccination amount as the previous immunization without adjuvant by intraperitoneal injection. One day before fusion, the feeder layer cells were prepared, and one BALB/c mouse aged 6-8 weeks was taken and killed by cervical dislocation after eyeball exsanguination. The dead BALB/c mouse was sterilized in 75% alcohol for 5 min and fixed on a plate, and the abdominal skin was cut aseptically in an ultra-clean table. 10 mL of hypoxanthine-aminopterin-thymidine selection (HAT) medium was absorbed by an aseptic syringe and injected into the abdominal cavity of the dead BALB/c mouse, followed by gently rubbing the abdomen with alcohol cotton balls, withdrawing the medium, and adding the withdrawn medium to 40 mL of HAT medium. The resulting medium was spread into four 96-well cell culture plates with 100 µL per well and incubated in a 5% CO₂ cell incubator at 37°C. One week before fusion, myeloma cells (Sp2/0 cells) were resuscitated, cultured in PRMI-1640 medium containing 10% fetal bovine serum, and subcultured in a 5% CO₂ incubator at 37°C. The cells in the logarithmic growth stage were collected into a centrifuge tube, counted, and diluted to 10⁷ cells/mL for later use. The BALB/c mouse immunized for 3 days was taken, and the positive serum was prepared by eyeball exsanguination. The mouse was killed by cervical dislocation and sterilized in 75% alcohol for 5 min, and the spleen was removed aseptically on the ultra-clean table. The spleen was washed several times on a sterile plate and the connective tissue was stripped. The resulting spleen was placed on a microporous copper net, and a fresh RPMI-1640 medium was added. The medium was absorbed by a syringe and injected into the spleen, and the spleen cells were blown down. After repeating the operations several times, the remaining spleen was gently ground with the inner plug of the syringe until there was no obvious red tissue mass. The spleen cell suspension from the plate was gently blown and transferred to a 50 mL centrifuge tube and centrifuged at 1000 r/min for 5 min to collect and count spleen cells for later use. The spleen cells of the immunized mouse and the Sp2/0 cells were mixed according to the cell number ratio of 10:1, added into a 50 mL centrifuge tube, and centrifuged at 1000 r/min for 5 min. The resulting supernatant was discarded, and the centrifuge tube was gently rubbed in the palm to make the two cells fully mixed. The centrifuge tube was placed in a 100 mL blue-capped bottle containing 37°C hot water. 1 mL of preheated dimethyl sulfoxide/polyethylene glycol (DMSO/PEG) was added into a fusion tube dropwise within 1 min, slowly at first and then quickly, and the centrifuge tube was gently rotated while adding the preheated DMSO/PEG. Subsequently, the reaction was terminated by immediately adding an antibody-free and blood-free RPMI-1640 medium at an amount of 1 mL for the first minute, 2 mL for the second minute, 3 mL for the third minute, and 4 mL for the fourth minute. The resulting product was placed in a water bath for 5 min at 37 °C and centrifuged at 800 r/min for 5 min, the resulting supernatant was discarded, and the resulting precipitate was suspended by HAT. The resulting mixture was mixed into 40 mL of a HAT selection medium containing 20% fetal bovine serum that was preheated at 37°C and spread into 96-well cell culture plates that was filled with feeder layer cells at a dose of 100 µL per well. The culture plates were put into a 5% CO₂ incubator at 37°C for incubation. Seven days later, a fresh HAT medium was used to replace half of the media in the culture plates. Ten days later, a thymidine (HT) medium was used to replace all media in the culture plates. The positive cells in the 96-well plates were subcloned by the limiting dilution method: First, the feeder layer cells were prepared according to the above method, and the hybridoma cells to be cloned were counted. The cells were diluted to 5-8 cells/mL with HT medium and added to the 96-well cell culture plates that were filled with feeder layer cells at a dose of 100 µL per well. Each hybridoma cell line was cloned into one 96-well cell culture plate and cultured in a 5% CO₂ cell incubator at 37°C. After about 5 days, the number of clones in the cell wells was counted and the clones were labeled. On the seventh day, the medium was replaced with a new medium. The detection was performed when 1/3 to 1/2 of the well bottom was covered by the cells. After all cell wells in the 96-well plates were positive by 2-3 times of cloning, an expanded culture could be carried out, followed by fixing and freezing the cell lines. The hybridoma cells that tested as positive were subjected to the expanded culture and freezing. The specific process is as follows: The hybridoma cells in vigorous growth and good condition were gently blown off the cell bottle with antibody-free and blood-free Dulbecco's modified eagle medium (DMEM) and centrifuged at 1000 r/min for 5 min. The resulting supernatant was discarded. A freezing medium (containing 40% RPMI-1640 medium, 50% fetal bovine serum, and 10% DMSO) was added, and the cells were blown out and sub-packaged into freezing tubes. The freezing tubes were placed in a freezing box and placed in a -70°C refrigerator. One day later, the freezing tubes were transferred to liquid nitrogen and recorded.

### 1.4 Preparation of ascites

Female BALB/c mice, aged 10-12 weeks, were intraperitoneally injected with sterile liquid paraffin at a dose of 0.5 mL/mouse. Seven days later, the hybridoma cells cultured to the logarithmic stage were intraperitoneally injected at a dose of 5×10⁶ cells/mouse. The mice were observed every day. About 7-10 days later, when the abdomen of the mice noticeably swelled, the lower abdominal skin was disinfected with 75% alcohol cotton balls, and a 16-gauge needle was used to penetrate the abdominal cavity to collect ascites. After the ascites was regenerated and accumulated, the ascites was collected again. The collected ascites was centrifuged at 3000 r/min for 10 min, and the clarified part in the middle was taken, followed by filtering through a filter paper, sub-packaging, and storing at -70°C.

### 1.5 Antibody purification

Ascites was purified using a Protein-G column through the following steps: 2 mL (n) of ascites was taken and centrifuged at 10000 g. The clarified part was taken and added with 2 mL (1:1) wash buffer for thorough mixing. The column was drained with 20% ethanol and equilibrated with 8 mL of wash buffer. The sample was passed through the column and settled at a flow rate of 8S/drop. The sample was repeatedly loaded 3 times, and then 15 mL of wash buffer was used to wash the precipitate at a flow rate of 8S/drop. After washing, 10 mL of elution buffer was used for elution. After elution, the resulting mixture was adjusted to have a pH of 7.4 with 1 M Tris having a pH of 9 concentrated with a concentrated column, and dialyzed overnight at 4°C in a dialysis bag filled with PBS.

### Embodiment 2: Identification of the anti-OXA-48-like carbapenemase mouse mAb

### 2.1 Identification of antibody subclasses

According to the instructions of the SIGMA kit, the subclass of mAb was identified by the ELISA capture method as follows: The mAb subclass identification reagent was diluted at 1:1000 and added into the enzyme-labeled wells at a dose of 100 µL per well. The resulting plates were incubated at 37°C for 1 h, washed 3 times with PBST, and pat for drying. The antibody was diluted at 1:1000 and added into the enzyme-labeled wells at a dose of 100 µL per well. The resulting plates were incubated at 37°C for 1 h, washed 3 times with PBST, and pat for drying. The goat anti-mouse immunoglobulin G-horseradish peroxidase (IgG-HRP) was diluted at 1:6000 and added at a dose of 100 µL per well. The resulting plates were incubated at room temperature for 30 min. The color development lasted for 10-20 min. The subclass type of the mAb was determined the same as that of subclass reagent added to the wells that had the maximum OD₄₅₀ value. The antibody subtype of the antibody IB5 was IgA, and the antibody subtype of the antibody IH3 was IgG1.

### 2.2 Antibody titer determination

The antibody titer was determined by an indirect ELISA method after purification through the following steps: The OXA-48-like carbapenemase was diluted to 0.2 µg/mL, 100 µL/well, and a non-coated control was set up. The resulting wells were coated at 4°C overnight, shaken for drying, and washed 3 times with PBST. Then the resulting wells were blocked at 37°C for 2 h with 5% skimmed milk powder at a dose of 200 µL per well, shaken for drying, and washed 3 times with PBST. Antibodies (initial concentration of 1 mg/mL) were subjected to doubling dilution starting from 1:1000 twelve times and each dilution was added at a dose of 100 µL per well. Meanwhile, a non-coated control was set up. The resulting wells were incubated at 37°C for 1 h, shaken for drying, and washed 3 times with PBST. Goat anti-mouse secondary antibody diluted in PBS at 1:6000 was added at a dose of 100 µL per well, incubated at 37°C for 45 min, shaken drying, and washed 5 times with PBST. TMB was added at a dose of 100 µL per well. Color development was performed at 37°C for 10 min. The reaction was terminated, and values were read. After purification, the antibody was diluted to 1 mg/mL, and the titer reached more than 1: 1280000.

### 2.3 Identification of purity and molecular weight of antibody

SDS-PAGE was used to identify the molecular weight and purity of the antibody. For gel preparation, the separated gel was 12%, and the concentrated gel was 5%. For sample preparation, 20 µL of sample and 20 µL of buffer were thoroughly mixed and boiled for 3 min. Each well was loaded with 20 µL of sample and a protein pre-staining marker control was set up. Electrophoresis was carried out at 80 V for 30 min and 120 V for 2 h. After the electrophoresis, the samples were stained with Coomassie brilliant blue solution. Decolorization was performed by boiling with deionized water for 5 min each time and 3 times in total. The molecular weight of the heavy chain of lgG antibody is generally 50-75 kDa, and the molecular weight of the light chain of the lgG antibody is about 25 kDa. As shown in FIG. 2, the purified mAb was identified by SDS-PAGE and the antibody IB5 and the antibody IH3 have clear bands at 50-75 kDa and about 25 kDa, respectively.

### Embodiment 3: Genetic validation of the anti-OXA-48-like carbapenemase mouse mAbs

Ig variable region genes were cloned by the reverse transcription-polymerase chain reaction (RT-PCR) method. Total RNA was extracted, and the single-stranded complementary DNA (cDNA) was synthesized.

Total RNA from IB5 and IH3 hybridoma cell lines was extracted using the Trizol method (kit purchased from Invitrogen) and reversed into cDNA libraries using Moloney murine leukemia virus (M-MLV) reverse transcriptase (purchased from Invitrogen).
Upstream primer of the heavy chain backbone region
P1: 5'SAGGTGMAGCTKCASSARTCWGG3', as shown in SEQ ID NO: 21;
Downstream primer of the heavy chain variable region
P2: 5'TGGGGSTGTYGTTTTGGCTGMRGAGACRGTGA3', as shown in SEQ ID NO: 22;
Upstream primer of the light chain precursor peptide
P3: 5'ATGGATTTTCAAGTGCAGATTTTCAG3', as shown in SEQ ID NO: 23;
Downstream primer of the light chain variable region
P4: 5' GGATACAGTTGGTGCAGCATCAGCCCGTTT3', as shown in SEQ ID NO: 24;

PCR reaction system (50 µL) was prepared as follows:
cDNA: 2 µL; upstream primer (10 µM): 2 µL; downstream primer (10 µM): 2 µL; dNTP mixture: 2 µL; pfu DNA polymerase (5 U/µL): 1 µL; 10×pfu Buffer II: 5 µL; and ddH₂O: supplementation to 50 µL.

Reaction conditions: Predenaturation at 95°C for 5 min; Thirty-five cycles of 95°C for 30 s, 58°C for 30 s, and 72°C for 1 min; Finally, extension at 72°C for 10 min.

The heavy chain variable region (VH) fragments and the light chain variable region (VL) fragments were separated and recovered by agarose gel electrophoresis. The recovered VL and VH fragments were ligated with T-Vector pMD19 (simple) (Takara Company), respectively, and the ligation system was as follows:
70 ng of the VL PCR product or 70 ng of the VH PCR product, respectively, 1 µL of the T-Vector pMD19 (simple), 5 µL of Solution I ligation reaction solution, and ddH₂O being supplemented to 10 µL; and ligation was performed overnight at 4°C.

The ligated product was transformed into *E.coli* DH5α competent bacteria and cultured overnight at 37°C. Subsequently, a single colony was selected and shaken at 37°C for 2 h. The bacterial liquid was identified by PCR, and the cDNA of the corresponding antibody was used as the positive control. The reaction system (25 µL) was prepared as follows:
Bacteria liquid: 1 µL; upstream primer (10 µM): 1 µL; downstream primer (10 µM): 1 µL; dNTP mixture (each 2.5 Mm): 2 µL; Taq DNA polymerase (5 U/µL): 0.5 µL; 10×Taq Buffer (Mg²⁺ plus): 2.5 µL; and water supplement to 25 µL. The reaction conditions were the same as the previous.

The PCR-positive clones were selected for expanded culture, and the positive clone plasmids were extracted by plasmid extraction kit (Takara Company) for sequencing. At least five cloned samples were sequenced for each chain of each antibody until at least three samples have the same sequencing results. The sequences of the heavy chain variable region and the light chain variable region of antibodies IB5 and IH3 were successfully cloned, which were consistent with the characteristics of typical antibody variable region sequences.

Embodiment 4: Preparation of OXA-48-like carbapenemase test card by the colloidal gold method

The double-antibody sandwich immuno-colloidal gold test strip was prepared as follows:
Step 1, A 0.1 M K₂CO₃ solution was added to a colloidal gold solution while stirring. After adjusting the pH value, the anti-OXA-48-like carbapenemase mouse mAb IH3 was added. After stirring, a 10% bovine serum albumin solution and 2% PEG 20000 were added. After stirring, the resulting mixture was centrifuged at a low speed to collect a supernatant, and then the resulting supernatant was centrifuged at a high speed to collect a precipitate. The colloidal gold resuspension was used to reach the predetermined volume to form a gold-labeled antibody.

Step 2, The gold-labeled antibody was sprayed on a glass cellulose film and dried to make a gold-labeled pad.

Step 3, The anti-OXA-48-like carbapenemase mouse mAb IB5 was added with a 1% thiomersal sodium solution and mixed well to form a test line coating solution. Then PBS and a 1% thiomersal sodium solution were added to the goat anti-mouse IgG and mixed well to form a quality control line coating solution. The quality control line coating solution and the test line coating solution were drawn on a nitrate cellulose film and dried to obtain a coated film.

Step 4, The coated film was attached to the bottom plate, the gold-labeled pad and the absorbent paper were attached to the coated film for lamination, and the resulting plate was cut to obtain the OXA-48-like carbapenemase test card by the colloidal gold method.

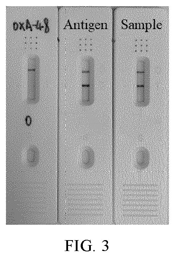

The OXA-48-like carbapenemase test card prepared by the colloidal gold method above was taken and spotted with a blank sample, an OXA-48-like carbapenemase, and a positive sample containing OXA-48-like carbapenemase, respectively. The results, as shown in FIG. 3 from left to right, are the blank sample, the OXA-48-like carbapenemase, and the positive sample containing OXA-48-like carbapenemase. It can be seen that the detection result of the blank sample is negative and the results of the OXA- 48 carbapenemase and the positive sample containing OXA-48-like carbapenemase are positive. It is proved that the OXA-48-like carbapenemase test card prepared by the colloidal gold method in the present solution can detect the OXA-48-like carbapenemase and the corresponding positive samples.

Embodiments of the present invention are described in detail above, but the contents are only preferred embodiments of the present invention and cannot be considered as limiting the scope of embodiments of the present invention.

## Claims

1. A detection reagent for a double-antibody sandwich immuno-colloidal gold test strip, comprising an anti-oxacillinase-48-like (OXA-48-like) carbapenemase mouse mAb IB5 and an anti-OXA-48-like carbapenemase mouse mAb IH3, wherein the antibody IB5 is used as a coated antibody, and the antibody IH3 is used as a gold-labeled antibody; or the antibody IH3 is used as the coated antibody, and the antibody IB5 is used as the gold-labeled antibody; **characterized in that**
the OXA-48-like carbapenemase mouse mAb IB5 is produced by an OXA-48-like carbapenemase mouse hybridoma cell line IB5 having an accession number of CGMCC No. 20284, wherein the antibody IB5 comprises a light chain variable region and a heavy chain variable region; the light chain variable region comprises CDRL1 as shown in SEQ ID NO: 1, CDRL2 as shown in SEQ ID NO: 2, and CDRL3 as shown in SEQ ID NO: 3; the heavy chain variable region comprises CDRH1 as shown in SEQ ID NO: 4, CDRH2 as shown in SEQ ID NO: 5, and CDRH3 as shown in SEQ ID NO: 6;
and,
the anti-OXA-48-like carbapenemase mouse mAb IH3 is produced by an OXA-48-like carbapenemase mouse hybridoma cell line IH3 having an accession number of CGMCC No. 20283, wherein the light chain variable region of the antibody IH3 comprises CDRL1 as shown in SEQ ID NO: 11, CDRL2 as shown in SEQ ID NO: 12, and CDRL3 as shown in SEQ ID NO: 13; the heavy chain variable region of the antibody IH3 comprises CDRH1 as shown in SEQ ID NO: 14, CDRH2 as shown in SEQ ID NO: 15, and CDRH3 as shown in SEQ ID NO: 16.

2. The detection reagent according to claim 1, wherein the amino acid sequence of the light chain variable region of the antibody IB5 is shown in SEQ ID NO: 7, and
the amino acid sequence of the heavy chain variable region of the antibody IB5 is shown in SEQ ID NO: 9; and,
the amino acid sequence of the light chain variable region of the antibody IH3 is shown in SEQ ID NO: 17, and the amino acid sequence of the heavy chain variable region is shown in SEQ ID NO: 19.

3. An *in vitro* diagnostic kit comprising the detection reagent according to claim 1, wherein the *in vitro* diagnostic kit is a colloidal gold immunoassay kit, a chemiluminescence kit, a radioimmunoassay kit, an enzyme-linked immunoassay kit, or a fluorescence immunoassay kit.

## Patentansprüche

1. Nachweisreagenz für einen Doppelantikörper-Sandwich-Immun-kolloidalen-Gold-Teststreifen, umfassend ein Anti-Oxacillinase-48-ähnliches (OXA-48-ähnliches) Carbapenemase-Maus-mAb IB5 und ein Anti-OXA-48-ähnliches Carbapenemase-Maus-mAb IH3, wobei der Antikörper IB5 als beschichteter Antikörper verwendet wird und der Antikörper IH3 als goldmarkierter Antikörper verwendet wird; oder der Antikörper IH3 als beschichteter Antikörper verwendet wird und der Antikörper IB5 als goldmarkierter Antikörper verwendet wird; **dadurch gekennzeichnet, dass** das OXA-48-ähnliche Carbapenemase-Maus-mAb IB5 durch eine OXA-48-ähnliche Carbapenemase-Maus-Hybridomzelllinie IB5 mit der Hinterlegungsnummer CGMCC Nr. 20284 hergestellt wird, wobei der Antikörper IB5 eine leichte Ketten-variable Region und eine schwere Ketten-variable Region umfasst; die leichte Ketten-variable Region umfasst CDRL1, gezeigt in SEQ ID NO: 1, CDRL2, gezeigt in SEQ ID NO: 2, und CDRL3, gezeigt in SEQ ID NO: 3; die schwere Ketten-Variable-Region umfasst CDRH1, gezeigt in SEQ ID NO: 4, CDRH2, gezeigt in SEQ ID NO: 5, und CDRH3, gezeigt in SEQ ID NO: 6;
und,
das die Anti-OXA-48-ähnliche Carbapenemase Maus-mAb IH3 durch eine OXA-48-ähnliche Carbapenemase Maus-Hybridomzelllinie IH3 mit einer Hinterlegungsnummer von CGMCC No. 20283 hergestellt wird, wobei die leichte Ketten-variable Region des Antikörpers IH3 CDRL1, gezeigt in SEQ ID NO: 11, CDRL2, gezeigt in SEQ ID NO: 12, und CDRL3, gezeigt in SEQ ID NO: 13 umfasst; die schwere Ketten-Variable-Region des Antikörpers IH3 umfasst CDRH1, gezeigt in SEQ ID NO: 14, CDRH2, gezeigt in SEQ ID NO: 15, und CDRH3, gezeigt in SEQ ID NO: 16.

2. Nachweisreagenz nach Anspruch 1, wobei die Aminosäuresequenz der leichten Ketten-variablen Region des Antikörpers IB5 in SEQ ID NO: 7 gezeigt ist, und
die Aminosäuresequenz der schweren Ketten-variablen Region des Antikörpers IB5 in SEQ ID NO: 9;
und,
die Aminosäuresequenz der leichten Ketten-variablen Region des Antikörpers IH3 in SEQ ID NO: 17 gezeigt ist, und die Aminosäuresequenz der schweren Ketten-variablen Region in SEQ ID NO: 19.

3. Ein *In-vitro*-Diagnostik-Kit, umfassend das Nachweisreagenz nach Anspruch 1, wobei das *In-vitro*-Diagnostik-Kit ein kolloidales-Gold-Immunoassay-Kit, ein Chemilumineszenz-Kit, ein Radioimmunoassay-Kit, ein Enzym-gekoppeltes-Immunoassay-Kit oder ein Fluoreszenz-Immunoassay-Kit ist.

## Revendications

1. Réactif de détection pour une bandelette d'essai immunologique à l'or colloïdal sandwich à double anticorps, comprenant un mAb de type oxacillinase-48 (de type OXA-48) carbapénémase de souris IB5 et un mAb de type OXA-48 carbapénémase de souris IH3, dans lequel l'anticorps IB5 est utilisé en tant qu'anticorps revêtu, et l'anticorps IH3 est utilisé en tant qu'anticorps marqué à l'or ; ou l'anticorps IH3 est utilisé en tant qu'anticorps revêtu, et l'anticorps IB5 est utilisé en tant qu'anticorps marqué à l'or ; **caractérisé en ce que** le mAb de type OXA-48 carbapénémase de souris IB5 est produit par une lignée cellulaire d'hybridome OXA-48 carbapénémase de souris IB5 ayant un numéro d'accès CGMCC n° 20284, dans lequel l'anticorps IB5 comprend une région variable à chaîne légère et une région variable à chaîne lourde ; la région variable à chaîne légère comprend CDRL1 telle que représentée dans SEQ ID n° : 1, CDRL2 telle que représentée dans SEQ ID n° : 2, et CDRL3 telle que représentée dans SEQ ID n° : 3 ; la région variable à chaîne lourde comprend CDRH1 telle que représentée dans SEQ ID n° : 4, CDRH2 telle que représentée dans SEQ ID n° : 5, et CDRH3 telle que représentée dans SEQ ID n° : 6 ;
et,
le mAb de type anti-OXA-48 carbapénémase de souris IH3 est produit par une lignée cellulaire d'hybridome de type OXA-48 carbapénémase de souris IH3 ayant un numéro d'accès CGMCC n° 20283, dans lequel la région variable à chaîne légère de l'anticorps IH3 comprend CDRL1 telle que représentée dans SEQ ID n° : 11, CDRL2 telle que représentée dans SEQ ID n° : 12, et CDRL3 telle que représentée dans SEQ ID n° : 13 ; la région variable à chaîne lourde de l'anticorps IH3 comprend CDRH1 telle que représentée dans SEQ ID n° : 14, CDRH2 telle que représentée dans SEQ ID n° : 15, et CDRH3 telle que représentée dans SEQ ID n° : 16.

2. Réactif de détection selon la revendication 1, dans lequel la séquence d'acides aminés de la région variable à chaîne légère de l'anticorps IB5 est représentée dans SEQ ID n° : 7, et
la séquence d'acides aminés de la région variable à chaîne lourde de l'anticorps IB5 est représentée dans SEQ ID n° : 9 ;
et,
la séquence d'acides aminés de la région variable à chaîne légère de l'anticorps IH3 est représentée dans SEQ ID n° : 17, et la séquence d'acides aminés de la région variable à chaîne lourde est représentée dans SEQ ID n° : 19.

3. Kit de diagnostic *in vitro* comprenant le réactif de détection selon la revendication 1, dans lequel le kit de diagnostic *in vitro* est un kit de dosage immunologique à l'or colloïdal, un kit de chimioluminescence, un kit de dosage radio-immunologique, un kit de dosage immunologique par enzyme liée, ou un kit de dosage immunologique par fluorescence.
